# EUROPEAN PATENT APPLICATION

(11) **EP 1 288 663 A1**
(43) Date of publication of application: **05.03.2003**
(21) Application number: 01982857.3
(22) Date of filing: 19.11.2001
(51) Int. Cl.: G01N 33/543

(54) **EXTRASOMATIC DIAGNOSTICS**

(30) Priority: 20.11.2000 JP 2000352210
(71) Applicant: MATSUSHITA ELECTRIC INDUSTRIAL CO., LTD., Kadoma-shi, Osaka 571-8501 (JP)
(72) Inventor: KITAWAKI, Fumihisa, Katano-shi, Osaka 576-0021 (JP); NADAOKA, Masataka, Iyo-shi, Ehime 799-3113 (JP); TAKAHASHI, Mie, Niihama-shi, Ehime 792-0026 (JP); TANAKA, Hirotaka, Matsuyama-shi, Ehime 791-1102 (JP); NAKAYAMA, Hiroshi, Hirakata-shi, Osaka 573-1114 (JP)
(74) Representative: Schwabe - Sandmair - Marx
(86) International application number: JP0110108
(87) International publication number: WO02040999

(57) **Abstract**

The present invention provides an extracorporeal diagnostic used for measurement of a substance to be tested in a specimen. The extracorporeal diagnostic comprises a reagent which specifically binds to the substance to be tested, and a hydrophilic material (a sugar or a sugar derivative). More particularly, the present invention relates to an extracorporeal diagnostic for measuring a substance to be tested in a specimen. The extracorporeal diagnostic comprises 1) a reagent which specifically binds to the substance to be tested and 2) a compound comprising at least one hydroxyl group and at least one aldehyde group or ketone group.

## Description

### TECHNICAL FIELD

The present invention relates to an extracorporeal diagnostic for use in a dry chemistry test method, and a diagnostic method. More particularly, the present invention relates to an extracorporeal diagnostic whose precision is maintained at a level no less than a predetermined level even after long-term storage, and a diagnostic method.

### BACKGROUND ART

Recently, a variety of test methods are utilized in clinical tests. One of the test methods is a dry chemistry test method. Dry chemistry is a method for measuring a substance to be tested in a sample, comprising spotting a liquid sample to be tested onto a reagent retained in dry form on a solid-phase matrix, such as film or litmus paper. Examples of the form of the extracorporeal diagnostic used in dry chemistry include a monolayer form in which a reagent is retained on a filter paper, a multilayer form in which a development layer, a reaction layer, a reagent layer, etc. are stacked on a layer, and the like. An exemplary characteristic feature of the dry chemistry test method is such that since a reagent is already retained on the solid-phase matrix, it is not necessary to prepare the reagent, the reagent can be stored in a space-saving manner, and only a small amount of sample to be tested is required.

A typical dry chemistry test method is, for example, immunochromatography. Immunochromatography is a test method utilizing an antigen-antibody reaction and capillary action. In an extracorporeal diagnostic used in this method, immobilized antibodies (or antigens) and antibodies (or antigens) sensitized to a detection reagent are each retained in dry form on a carrier, such as a membrane filter or the like. In testing, a sample to be tested containing antigens (or antibodies) is added onto the extracorporeal diagnostic, and is developed by capillary action, causing a sandwich-type antigen-antibody reaction at a reaction site. Thereafter, a color is caused to be developed at the reaction site so as to identify an antigen (or an antibody) in a sample to be tested, or determine the presence or absence or the amount of the antigen (or the antibody). The form of the antigen-antibody reaction includes a competition-type reaction in addition to the sandwich-type reaction. Immunochromatography may utilize the competition-type reaction. In this case, the structure of the device and the test method are similar to those in sandwich-type immunochromatography. An immunochromatographic device utilizing such an immunochromatography principle is used mainly for a qualitative determination to determine a positive or negative result depending on the presence or absence of a coloration at a reaction site, as exemplified by a pregnancy test device.

The advantages of a test method utilizing immunochromatography include ease of handling, quick determination, and low cost, in addition to the above-described advantages of the dry chemistry. Therefore, the test method utilizing immunochromatography is not limited to clinical tests and is applicable to point of care (hereinafter abbreviated as POC) which has recently received attention. Particularly, in the scene of medical diagnosis based on the concept of POC, handling of the reagent is an important matter. With respect to this point, an immunochromatographic device for use in qualitative determination which can be stored at room temperature is easy for the user to handle, and handling the device does not require expert knowledge or techniques for the reagent retained on the device, such as an antigen, an immobilized antibody, a labeled antibody, or the like.

Moreover, recently, immunochromatography has been utilized for semi-quantitative or quantitative determination by measuring the density of a coloration at a reaction site. Means for semi-quantification or quantification include a method for measuring absorbance at a reaction site typically using a reflection-absorbance method. Quantitative determination can clarify a variety of medical matters which cannot be revealed by qualitative determination. Therefore, attention has been focused on the usefulness of quantitative determination.

However, differently from qualitative determination based on the presence or absence of a coloration, semi-quantitative or quantitative determination is performed based not only on the presence or absence of a coloration but also on the density of the coloration. Therefore, the semi-quantitative or quantitative determination requires a higher level of storage stability. Conventional quantitative immunochromatographic devices can provide high-precision quantitative determination immediately after production thereof, but have a drawback that the high-precision quantification capability cannot be maintained after long-term storage. The maintenance of the high-precision quantification capability requires that development of a sample into a carrier is uniform over time and dissolution of a labeled antibody is uniform over time.

As a specific strategy for maintaining the stability of immunochromatographic devices, bovine serum albumin (hereafter abbreviated as BSA) and dextrin have been added as stabilizers for the purpose of preventing reduction in the affinity of an immobilized antibody which is an important factor of the device, as disclosed in Japanese Patent No. 1849714. However, this method did not provide a sufficient level of storage stability to allow quantification determination.

Moreover, among dry chemistry-based extracorporeal diagnostics other than immunochromatographic devices, there are high-precision quantification devices, but such devices have poor storage stability and a difficulty in using for POC.

In view of the above-described problems, the object of the present invention is to provide an extracorporeal diagnostic having excellent storage stability in quantitative determination as well.

### DISCLOSURE OF THE INVENTION

In order to solve the above-described problems, the present invention provides an extracorporeal diagnostic for measuring a substance to be tested in a specimen. The extracorporeal diagnostic is characterized by comprising a reagent which specifically binds to the substance to be tested, and a hydrophilic material (e.g., a sugar or a sugar derivative).

The present invention provides the following.
(1) An extracorporeal diagnostic for measuring a substance to be tested in a specimen, comprising:
   1) a reagent which specifically binds to the substance to be tested; and
   2) a compound comprising at least one hydroxyl group and at least one aldehyde group or ketone group.
(2) An extracorporeal diagnostic according to (1), wherein the reagent is an antibody or an antigen, or a derivative thereof.
(3) An extracorporeal diagnostic according to (1), wherein the compound is a sugar or a sugar derivative.
(4) An extracorporeal diagnostic according to (1), further comprising bovine serum albumin, casein, surfactant, or skim milk.
(5) An extracorporeal diagnostic according to (1), wherein the compound comprises sugar alcohol or a derivative thereof.
(6) An extracorporeal diagnostic according to (1), wherein the compound comprises sucrose or sorbitol.
(7) An extracorporeal diagnostic according to (1), wherein the compound comprises sucrose and sorbitol.
(8) An extracorporeal diagnostic according to (1), wherein the compound is present at a concentration of no less than about 3 w/v%.
(9) An extracorporeal diagnostic according to (1), wherein the compound is present at a concentration of about 3 w/v% to about 10 w/v%.
(10) An extracorporeal diagnostic according to (1). wherein the extracorporeal diagnostic comprises a plurality of reagents which specifically bind to the protein, the plurality of reagents comprise a first antibody or antigen, or a derivative thereof, and a second antibody or antigen, or a derivative thereof.
(11) An extracorporeal diagnostic according to (1), wherein the reagent is labeled with a colloidal particle, a latex particle, a pigment, a micelle, an enzyme, a fluorescent material, or a phosphorescent material.
(12) An extracorporeal diagnostic according to (1), wherein the reagent and the compound are retained on a support.
(13) An extracorporeal diagnostic according to (12), wherein the reagent and the compound are retained on separate regions of the support.
(14) An extracorporeal diagnostic according to (12), wherein the support is a solid-phase matrix.
(15) An extracorporeal diagnostic according to (12), wherein the support comprises a porous material.
(16) An extracorporeal diagnostic according to (12), wherein the support comprises a labeling region, a determination region, and a sample introduction region,
   1) the labeling region comprises a first antibody or antigen which binds to the substance to be tested,
   2) the determination region comprises a second antibody or antigen which binds to the substance to be tested, and is disposed in fluid communication with the labeling region,
   3) the sample introduction region is disposed in fluid communication with the labeling region.
(17) An extracorporeal diagnostic according to (16), wherein the compound is contained in at least one region selected from the group consisting of the labeling region and the determination region.
(18) An extracorporeal diagnostic according to (16), wherein the compound is contained in the labeling region.
(19) A method for producing an extracorporeal diagnostic for measuring a substance to be tested in a specimen, the method comprising the steps of:
   1) providing a reagent which specifically binds to the substance to be tested; and
   2) providing a compound comprising at least one hydroxyl group and at least one aldehyde group or ketone group.
(20) A method for detecting a substance to be tested in a specimen, the method comprising the steps of:
   A) providing an extracorporeal diagnostic, the extracorporeal diagnostic comprising:
      1) a reagent which specifically reacts with the substance to be tested; and
      2) a compound comprising at least one hydroxyl group and at least one aldehyde group or ketone group;
   B) providing the specimen to the extracorporeal diagnostic;
   C) disposing the extracorporeal diagnostic under a condition that the reagent specifically reacts with the specimen; and
   D) detecting a signal caused by a specific reaction of the reagent with the specimen.
(21) Use of an extracorporeal diagnostic for measuring a substance to be tested in a specimen, the extracorporeal diagnostic comprising:
   1) a reagent which specifically binds to the substance to be tested; and
   2) a compound comprising at least one hydroxyl group and at least one aldehyde group or ketone group.

### BRIEF DESCRIPTION OF THE DRAWINGS

Figure **1** is a perspective view showing an immunochromatographic device according to an embodiment of the present invention.
Figure **2** is a graph showing the storage stability at 4°C of the immunochromatographic device of an embodiment of the present invention.
Figure **3** is a graph showing the storage stability at 4°C of the immunochromatographic device of a comparative example.
Figure **4** is a graph showing the storage stability at 25°C of the immunochromatographic device of an embodiment of the present invention.
Figure **5** is a graph showing the storage stability at 25°C of the immunochromatographic device of the comparative example.
Figure **6** is a graph showing the storage stability at 40°C of the immunochromatographic device of an embodiment of the present invention.
Figure **7** is a graph showing the storage stability at 40°C of the immunochromatographic device of a comparative example.
Figure **8** is a graph showing degradation of the intensity of a coloration for various sucrose concentrations due to storage at 40°C for one month.
Figure **9** is a diagram showing the storage stability at 25°C of the sensitivity of an immunochromatographic device according to Example 3.
Figure **10** is a diagram showing the precision (CV value) of the storage stability at 25°C of the immunochromatographic device of Example 3.
Figure **11** is a diagram showing the storage stability at 25°C of an immunochromatographic device of a comparative example.
Figure **12** is a diagram showing the precision (CV value) of the storage stability at 25°C of the immunochromatographic device of a comparative example.
Figure **13** is a diagram showing stability indexes representing the degradation rates of the example of the present invention (Figure **9**), where measured values at day 14 are used as a reference.
Figure **14** is a diagram showing stability indexes representing the degradation rates of the comparative example (Figure **11**), where measured values at day 14 are used as a reference.

### (Description of Reference Numerals)

**10** Sample introduction region
**11** Labeling region
**12** Determination region
**13** Substrate

### BEST MODE FOR CARRYING OUT THE INVENTION

It should be understood throughout the present specification that articles for a singular form (e.g., "a", "an", "the", etc. in English; "ein", "der", "das", "die". etc. and their inflections in German; "un", "une", "le", "la", etc. in French; and articles, adjectives, etc. in other languages) include the concept of their plurality unless otherwise mentioned. It should be also understood that the terms as used herein have definitions typically used in the art unless otherwise mentioned.

The present invention provides an extracorporeal diagnostic for measuring a substance tobe tested in a specimen. The extracorporeal diagnostic comprises:
1) a reagent which specifically reacts with the substance to be tested; and
2) a hydrophilic material.

"Extracorporeal diagnostic" as used herein refers to a product which can be monitored from the outside of the body of a subject on at least one specific biological parameter. The extracorporeal diagnostic may be in any form depending on the situation, e.g., may be in the form of a composition or a device.

"Hydrophilic material" as used herein refers to a material comprising an atom group having a strong affinity for water molecules. It is herein necessary for the hydrophilic material not to destroy the three-dimensional structure of proteins. Examples of such a hydrophilic material include a side chain of an amino acid, such as lysine, arginine, glutamic acid, aspartic acid, and the like; the phosphate group of a nucleic acid; a side chain of an amino acid, such as serine, threonine, and the like; the hydroxyl group of a sugar or a sugar derivative; and the like.

In one embodiment, the above-described hydrophilic material comprises a compound comprising at least one hydroxyl group and at least one aldehyde group or ketone group. Therefore, in a preferred embodiment, the present invention provides an extracorporeal diagnostic for measuring a substance to be tested in a specimen. The extracorporeal diagnostic comprises:
1) a reagent which specifically reacts with the substance to be tested; and
2) a compound comprising at least one hydroxyl group and at least one aldehyde group or ketone group.

In one preferred embodiment, the above-described hydrophilic material may be a sugar or a sugar derivative.

Sugar as used herein refers to polyhydroxyaldehyde or polyhydroxyketone comprising at least one hydroxyl group and at least one aldehyde group or ketone group. As used herein, sugar also refers to carbohydrate, and both are interchangeably used.

Sugar used for the extracorporeal diagnostic of the present invention may be any sugar that can be dissolved in liquid. Examples of such a sugar include monosaccharides, such as glucose, mannose, galactose, fructose, and the like, and oligosaccharides, such as maltose, isomaltose, cellobiose, lactose, sucrose, and the like. Further, the sugar may include polysaccharides in which monosaccharides or oligosaccharides are chemically linked together. The sugar has stereoisomers. All of the stereoisomers can be applied to the extracorporeal diagnostic of the present invention. Among the sugars, sucrose is preferable.

A sugar derivative as used herein refers to a sugar whose substituents are substituted with other substituents, and a sugar variant obtained by an oxidation-reduction reaction of a sugar. Here, the substituent includes, but is not limited to, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, ciano, nitro, amino, substitutedamino, carboxy, substituted carboxy, acyl, substituted acyl, thiocarboxy, substituted thiocarboxy, amide, substituted amide, substituted carbonyl1 substituted thiocarbonyl, substituted sulfonyl and substituted sulfinyl.

A sugar derivative used for the extracorporeal diagnostic of the present invention may be any sugar derivative which can be dissolved in liquid. Examples of the sugar derivative include: components of organisms, such as proteins, lipids, nucleic acids, and the like, modified with monosaccharides, oligosaccharides or polysaccharides; sugar alcohol; inositol; uronic acid; ascorbic acid; amino sugar; sugar phosphate ester; naturally occurring glycoprotein; and the like. Among these sugar derivatives, sugar alcohol is preferable.

Sugars or sugar derivatives have hygroscopicity, a low level of drying property, and an excellent level of moisture retention property against temperature changes. In this case, the moisture content of the extracorporeal diagnostic can be moderately retained. Therefore, it is possible to obtain an extracorporeal diagnostic having storage stability with respect to high-precision quantitative determination.

A sugar alcohol, which can be used in the extracorporeal diagnostic of the present invention, includes chain polyhydric alcohol obtained by reducing the carbonyl group of aldose or ketose, or stereoisomers thereof, including, for example, glycerol, erythritol, threitol, ribitol, arabinitol, xylitol, allitol, sorbitol, mannitol, iditol, dulcitol and talitol, or, the stereoisomers of glycerol, erythritol, threitol, ribitol, arabinitol, xylitol, allitol, sorbitol, mannitol, iditol, dulcitol and talitol. Further, the sugar alcohol may include a compound in which at least two of the above-described chain polyhydric alcohols or stereoisomers thereof, are chemically linked together, including, for example, a compound in which two of glycerol, erythritol, threitol, ribitol, arabinitol, xylitol, allitol, sorbitol, mannitol, iditol, dulcitol or talitol, or D- and L-stereoisomers of glycerol, erythritol, threitol, ribitol, arabinitol, xylitol, allitol, sorbitol, mannitol, iditol, dulcitol or talitol, are chemically linked together. Furthermore, the sugar alcohol may include a compound in which the above-described chain polyhydric alcohols or stereoisomers thereof, including, for example, glycerol, erythritol, threitol, ribitol, arabinitol, xylitol, allitol, sorbitol, mannitol, iditol, dulcitol or talitol, or stereoisomers of glycerol, erythritol, threitol, ribitol, arabinitol, xylitol, allitol, sorbitol, mannitol, iditol, dulcitol or talitol, are partially or entirely linked with naturally occurring sugar or non-naturally occurring and artificially synthesized sugar, or stereoisomers of naturally occurring sugar or non-naturally occurring and artificially synthesized sugar via an alcohol group. Among them, sugar alcohol is preferably sorbitol.

Therefore, preferably, the above-described material may comprise sugar alcohol or derivatives thereof. More preferably, the above-described material may comprise sucrose or sorbitol. It may be preferable that the above-described material comprises sucrose and sorbitol.

In one embodiment, the above-described material may be present at a concentration of more than about 1 w/v%. More preferably, the above-described material is present at a concentration of at least about 3 w/v%. Even more preferably, the above-described material is present at a concentration of about 3 w/v% to about 10 w/v%. The content concentration of the above-described material is not limited to these ranges, and includes, for example, about 1 w/v%, about 1.5 w/v%, about 2 w/v%, about 2.5 w/v%, about 3 w/v%, about 4 w/v%, about 5 w/v%, about 6 w/v%, about 7 w/v%, about 8 w/v%, about 9 w/v%, about 10 w/v%, about 15 w/v%, and the like as a lower limit. The upper limit of the content concentration of the above-described material includes, but is not limited to, for example, about 1.5 w/v%, about 2 w/v%, about 2.5 w/v%, about 3 w/v%, about 4 w/v%, about 5 w/v%, about 6 w/v%, about w/v%, about 8 w/v%, about 9 w/v%, about 10 w/v%, about 12.5 w/v%, about 15 w/v%, about 17.5 w/v%, about 20 w/v%, about 30 w/v%, about 40 w/v%, about 50 w/v%, and the like. The above-described range may be any combination of the above-described lower and upper limits.

A reagent which can be used in the present invention, is any reagent which can specifically react with a substance to be tested, including, for example, antibodies, antigens, avidin, biotin, nucleic acids, and the like. Among them, antigens or antibodies are preferable. Therefore, in another aspect, the reagent used in the present invention may be an antibody or antigen, or a derivative thereof. "Specifically react with" refers to an interaction with an intended object which is stronger than an interaction with other objects.

"Antigen" as used herein refers to any substance which raises an antibody. Such an antigen includes haptens, proteins, bacteria, viruses, anti-viral antibodies, and the like. Here, examples of the haptens include low molecular weight compounds, such as dioxin, amphetamine, methamphetamine, estradiol, and the like. The proteins include hemoglobin, albumin, hemoglobin Alc (glycohemoglobin), HDL (high density lipoprotein), LDL (low density lipoprotein), HCV antibody (hepatitis C virus antibody), HIV antibody (human immunodeficiency virus antibody), CEA (carcinoembryonic antigen), AFP (α-fetoprotein), CRP (C responsive protein), SAA (serum amyloid A) , hCG (human chrionic gonadotropin), and the like. The bacteria include bacteria belonging to the genus E. coli, the genus Salmonella, the genus Staphylococcus aureus, or the genus Vibrio. The viruses include HIV, HBs, and the like.

"Antibody" as used herein comprises an antibody raised by the above-described antigens. The antibody is also herein intended to include the entirety of the antibody, and an immunogenic derivative and fragment thereof. The immunogenic fragment of the antibody refers to any fragment of the antibody having immunogenicity. Examples of the immunogenic fragment of the antibody include, but are not limited to, variable regions, such as Fab, F(ab)₂' and the like. Therefore, the immunogenic fragment of the antibody may be any fragment as long as it can raise ah immune reaction.

In another embodiment, the extracorporeal diagnostic of the present invention may further comprise bovine serum albumin, casein, surfactant, or skim milk.

In the extracorporeal diagnostic of the present invention in one aspect, the extracorporeal diagnostic comprises a plurality of the above-described reagents. The at least two reagents may comprise a first antibody or antigen, or a derivative thereof, and a second antibody or antigen, or a derivative thereof.

In a preferred embodiment, the above-described reagent may be labeled with a colloidal particle, a latex particle, a pigment, a micelle, an enzyme, a fluorescent material or a phosphorescent material.

In one embodiment, the above-described reagent and the above-described material may be retained on a support. Preferably, the above-described reagent and the above-described compound may be retained on other regions of the above-described support.

In a preferred embodiment, the above-described support may be a solid-phase matrix. The matrix may be in the form of a layer (solid-phase matrix). Preferably, the above-described support may comprise a porous material. Here, preferably, the reagent and a sugar or a sugar derivative are retained on the solid-phase matrix.

In general, the solid-phase matrix is any solid-phase matrix which can absorb, retain and develop sample solution, and can adsorb a biological material physically or chemically, including, for example, glass fiber filter paper, membrane filter, and the like.

The extracorporeal diagnostic of the present invention comprises a sample introduction region, a labeling region containing a first antibody or antigen which binds to an antigen or antibody which is a substance to be tested, and a determination region containing a second antibody or antigen which binds to the antigen or antibody which is a substance to be tested, as a solid-phase matrix. The extracorporeal diagnostic is characterized in that: the sample introduction region, the labeling region and the determination region are arranged so that the sample solution is introduced into the sample introduction region and is then transferred via the labeling region to the determination region; the sample introduction region, the labeling region or the determination region contains a sugar or a sugar derivative; and an antigen or antibody in the sample solution, which is a substance to be tested, is measured based on a specific binding reaction between antigen and antibody.

Thereby, the moisture content of at least the sample introduction region, the labeling region or the determination region is appropriately retained, so that development of the sample solution to the carrier is uniform over time. Therefore, it is possible to obtain an extracorporeal diagnostic having storage stability with respect to high-precision quantitative determination.

In a preferred embodiment, in the extracorporeal diagnostic of the present invention, the above-described support comprises a labeling region, a determination region and a sample introduction region,
1) the labeling region contains the first antibody or antigen which binds to the substance to be tested,
2) the determination region contains the second antibody or antigen which binds to the substance to be tested, and is disposed in fluid communication with the labeling region,
3) the sample introduction region is disposed in fluid communication with the labeling region.

In a preferred embodiment, the extracorporeal diagnostic of the present invention may be, but is not limited to, in the form of an extracorporeal diagnostic device. Therefore, the extracorporeal diagnostic may also be in the form of a composition.

Here, the labeling region preferably contains a sugar or a sugar derivative. In this case, adsorption of the first antibody (or antigen) into the solid-phase matrix is relaxed, whereby dissolution of the first antibody (or antigen) is uniform over time, and an extracorporeal diagnostic having a higher level of storage stability can be obtained.

In one embodiment, the first antibody (or antigen) may be labeled with a colloidal particle, a latex particle, a pigment, or a micelle, and at least the labeling region preferably contains a sugar or a sugar derivative.

A material for the sample introduction region may be any material which can develop the sample solution at an appropriate speed, including, for example, nitrocellulose and glass filter paper.

Moreover, a material for the labeling region and the determination region may be any material which can retain the first and second antibodies (or antigens) and develop the sample solution at an appropriate speed, including, for example, nitrocellulose and glass filter paper.

In one embodiment of the present invention, for example, an extracorporeal diagnostic is provided, which is required when a substance to be tested is measured using an automatic analyzer and which is prepared by adding a sugar. or a sugar derivative to a liquid reagent contained in a cuvette, an Eppendorf tube, or the like, followed by drying. A variety of applications are expected when such an extracorporeal diagnostic is used in measurement. For example, a buffer solution or the like is poured into the above-described container containing the lyophilized product to dissolve the lyophilized product and thereafter, the resultant solution is transferred to a cuvette for the analyzer so that the solution can be measured. Alternatively, the container containing the solution can be directly placed in the analyzer and the analyzer can automatically pour a buffer solution or the like into the container so that measurement can be immediately started. With the dried reagent-containing container, the user does not have to prepare a reagent. Further, since the reagent is in a solid state, the reagent is easy to handle unlike when it is in a liquid state. If the container is disposable, washing the container is not required, thereby expecting that handling the container is easier. Furthermore, the container can be stored at room temperature, thereby expecting that handling the container is even easier.

In another embodiment of the present invention, an extracorporeal diagnostic is provided, which is used in utilizing an immune serum test method, such as immunonephelometry, nephelometry, latex agglutination test, radioimmunosorbent test, enzyme-linked immunosorbent assay, immunofluorescence assay, chemiluminescence immunoassay, particle coefficient immunoassay, and the like, and which is prepared by adding a sugar or a sugar derivative to an antibody or antigen solution contained in a cuvette, an Eppendorf tube, or the like, followed by lyophilization. The extracorporeal diagnostic can be handled in a manner similar to the above-described usage of the automatic analyzer when a reagent is handled and measured.

In one embodiment, the above-described compound may be contained in at least one region selected from the group consisting of the labeling region and the determination region. Preferably, the compound may be contained in at least the labeling region, and may be contained in all of the regions.

In one aspect, the present invention provides a method for producing an extracorporeal diagnostic for measuring a substance to be tested in a specimen. The method comprises:
1) providing a reagent which specifically reacts with the substance to be tested; and
2) providing a compound comprising at least one hydroxyl group and at least one aldehyde group or ketone group.

In one embodiment, the extracorporeal diagnostic of the present invention can be prepared by mixing a solution of a reagent which specifically reacts with a substance to be tested with a sugar or a sugar derivative, and air drying or lyophilizing the solution.

When a reagent and a sugar or a sugar derivative are retained on a solid-phase matrix, a solution of a reagent which specifically reacts with a substance to be tested is mixed with a sugar or a sugar derivative, and the resultant solution is caused to permeate or be immobilized to the solid-phasematrix, followed by air drying or lyophilization. It should be noted that "permeate" herein indicates that the reagent is retained in such a manner that the reagent can be dissolved, and "immobilized" herein indicates that the reagent is retained in such a manner that the reagent cannot be dissolved.

Further, the extracorporeal diagnostic of the present invention can be prepared by mixing a solution containing a first antibody (or antigen) with a sugar or a sugar derivative, causing the resultant solution to permeate the labeling region, or/and mixing a solution containing a second antibody (or antigen) with a sugar or a sugar derivative, and causing the resultant solution to be immobilized in the determination region, followed by air drying or lyophilization.

In another aspect, the present invention provides a method for detecting a substance to be tested in a specimen. The method comprises the steps of:
A) providing an extracorporeal diagnostic, the extracorporeal diagnostic comprising:
   1) a reagent which specifically reacts with the substance to be tested; and
   2) a compound comprising at least one hydroxyl group and at least one aldehyde group or ketone group;
B) providing the specimen to the extracorporeal diagnostic;
C) disposing the extracorporeal diagnostic under a condition that the reagent specifically reacts with the specimen; and
D) detecting a signal caused by a specific reaction of the reagent with the specimen.

In another aspect, the present invention relates to use of the extracorporeal diagnostic of the present invention for measuring the substance to be tested in the specimen.

With the diagnostic method or detection method of the present invention, it is possible to detect, for example, components in blood (e.g., HbAlc (glycohemoglobin), HDL (high density lipoprotein), LDL (low density lipoprotein), HCV antibody (hepatitis C virus antibody), HIV antibody (human immunodeficiency virus antibody), CEA (carcinoembryonic antigen), AFP (α-fetoprotein), CRP (C responsive protein), SAA (serum amyloid A), etc.). To this end, for example, blood is spotted onto an immunochromatographic device which is used to detect or immobilize an antibody against the above-described proteins, and the intensity of a coloration after a predetermined time is determined by measuring reflection absorbance, thereby making it possible to quantify the concentration of the proteins.

The effects of the present invention include that high-precision diagnosis and detection can be achieved after long-term storage (e.g., one month). "Stability index" is herein used as an index for evaluating precision after a predetermined time. "Stability index" as used herein refers to the sensitivity of an extracorporeal diagnostic relative to a reference where the sensitivity of the extracorporeal diagnostic 14 days after production thereof is the reference (0). Therefore, when a 14-day sensitivity is -10 and a 30-day sensitivity is 20, a corresponding stability index is +30. It is desirable that the stability index is close to 0. Further, it is also preferable that a variation in the stability index at each measurement time point is small, i.e., the stability index does not vary much with time. In the present invention, the value of the stability index may be preferably within ±20. More preferably, the stability index may be within ±10. If the variation is within ±20, since variations in the device have a larger influence, the variation of the stability index cannot be simply said to be caused by degradation. Such a range may be tolerable. In another aspect, in the present invention, it is preferable that the difference between the stability indexes at measurement time points is within 20. More preferably, such a difference may be within 10. When the storage stability is evaluated, it is important to predict a result of a subsequent measurement concerning measured data. In a storage stability test, it is also important to predict a result of measurement after a final measurement time point. Therefore, it is desirable that the stability index does not vary much with time, and particularly does not continuously vary to a large extent. Conversely, when the stability index for a certain concentration is beyond the range within ±20 and the absolute value of the stability index tends to increase with an increase in time, it can be said that the possibility that subsequent measurement produces unreliable data is high. Moreover, it is expected that the absolute value of the stability index is beyond 20 with respect to other concentrations. Actually, a similar tendency was observed in comparative examples herein described.

Therefore, the present invention can provide high-precision diagnosis and detection after long-term storage including, but not limited to, for example, one month, two months, three months, six months, one year, and the like.

Hereinafter, the present invention will be described in more detail by way of examples. The examples below are provided only for illustration purposes. Therefore, the scope of the present invention is limited only by the claims, but not by the examples.

### EXAMPLES

Reagents, instruments, and systems used in the examples below are those commonly used in the art. It should be noted that an extracorporeal diagnostic will be described, exemplifying an immunochromatographic device. The present invention is not limited to the examples below.

### (Example 1)

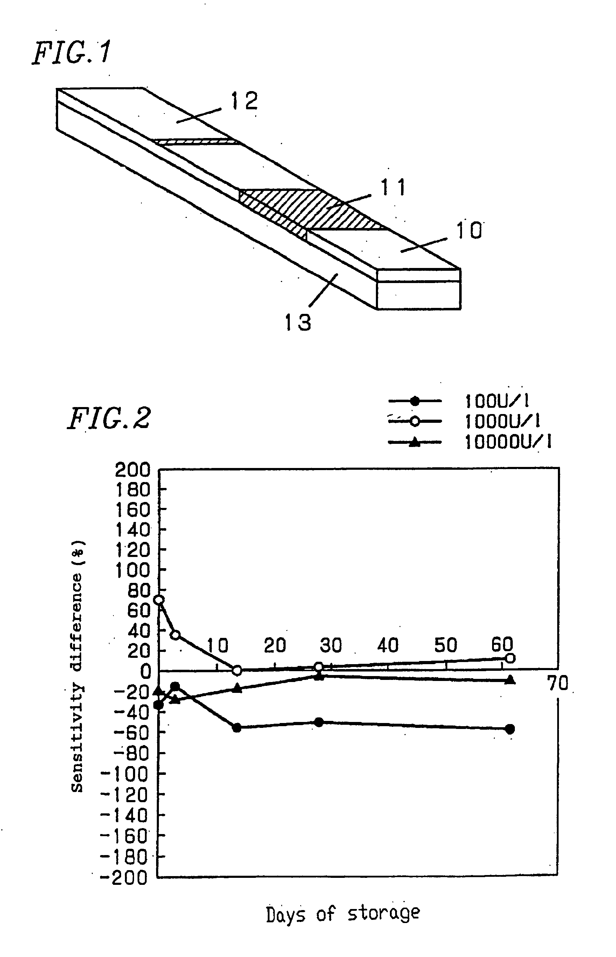

An immunochromatographic device for quantitatively measuring hCG in human urine was prepared, in which human urine and hCG were used as a sample solution and an antigen, i.e., a substance to be tested, respectively, and sorbitol, which is a sugar alcohol, was contained in a labeling region. The storage stability of the device was assessed. Figure 1 shows a structure of an immunochromatographic device according to Example 1.

### (Preparation of nitrocellulose membrane)

The concentration of anti-hCG-β antibody as a second antibody was adjusted using phosphate buffer solution (pH 7.4) and thereafter, the resultant anti-hCG-β antibody solution was applied using a solution discharging apparatus onto a central region of a 150-µm thick nitrocellulose membrane (manufactured by Millipore: high flow membrane) into line, followed by drying. Thus, a determination region **12** was prepared. Next, nitrocellulose membrane was immersed in Tris(hydroxymethyl)aminomethane (hereinafter referred to as Tris) - HCl buffer solution (pH 8.2) containing 1% skim milk for 30 minutes while shaking and thereafter, was immersed in another Tris-HCl buffer solution (pH 8.2) for 10 minutes while shaking. The thus-obtained nitrocellulose membrane was dried at room temperature.

### (Preparation of gold colloid sensitized anti-hCG-α antibody solution)

Initially, 4 ml of 1% citric acid solution was quickly added to 200 ml of refluxing 0.01% chloroauric acid solution, thereby producing gold colloid. The gold colloid solution was cooled at room temperature and was thereafter adjusted to pH 9.0 by adding 0.2 M potassium carbonate solution. Next, 500 µl of PBS buffer solution containing 5 mg/ml anti-hCG-α antibody was added as a first antibody, followed by shaking for several minutes. Further, 20 ml of 10% bovine serum albumin (hereinafter referred to as BSA) solution (pH 9.0) was added. The thus-obtained reaction mixture containing the gold colloid sensitized anti-hCG-α antibody was centrifuged twice so as to remove unreacted anti-hCG-α antibody and BSA. The thus-purified gold colloid sensitized anti-hCG-α antibody was suspended in phosphate buffer solution (pH 7.4) containing 10% sorbitol and passed through a 0.8-µm filter (manufactured by ADVANTEC: DISMIC-25cs), and stored at 4°C.

### (Preparation of immunochromatographic device)

The prepared gold colloid sensitized anti-hCG-α antibody solution was applied using a solution discharging apparatus onto a portion of a position away from the determination region **12** of the above-described nitrocellulose membrane, followed by drying, thereby producing a labeling region **11** on the nitrocellulose membrane. Further, a portion of the nitrocellulose membrane, onto which the gold colloid sensitized anti-hCG-α antibody solution was not applied, was referred to as a sample introduction region **10**.

Finally, the above-described nitrocellulose membrane was attached to a substrate **13** of white PET having a thickness of 0.5 mm, and cut into 5 mm × 50 mm strips, thereby producing immunochromatographic devices.

An immunochromatographic device, in which sorbitol was not contained in a labeling region, was produced in the same manner as that in Example 1, except that sorbitol was not used in the step of preparing a gold colloid sensitized anti-hCG-α antibody solution.

### (Preparation of sample solution)

hCG solution having a known concentration was added to human urine to adjust the concentration thereof to 100 U/l, 1000 U/l and 10000 U/l.

### (Quantitative determination of hCG concentration)

Quantitative determination of hCG concentration in sample solution was performed immediately after production of an immunochromatographic device and 3 days, 14 days, 28 days and 62 days after the production for the immunochromatographic device of Example 1, and immediately after production of an immunochromatographic device and 7 days, 14 days, 28 days and 62 days after the production for the immunochromatographic device of Comparative Example 1. For this period, the immunochromatographic devices were stored at 4°C, 25°C and 40°C for both Example 1 and Comparative Example 1.

In measurement, 40 µl of 100 U/l, 1000 U/l or 10000 U/l hCG solution was added to the sample introduction region **10** of the immunochromatographic device prepared and was allowed to develop on the membrane. Quantification of the hCG concentration of a sample solution was performed after 5 minutes by measuring a coloration in the determination region **12** by reflectance absorbance at 520 nm using a reflection absorption spectrophotometer (manufactured by Shimadzu: CS9300).

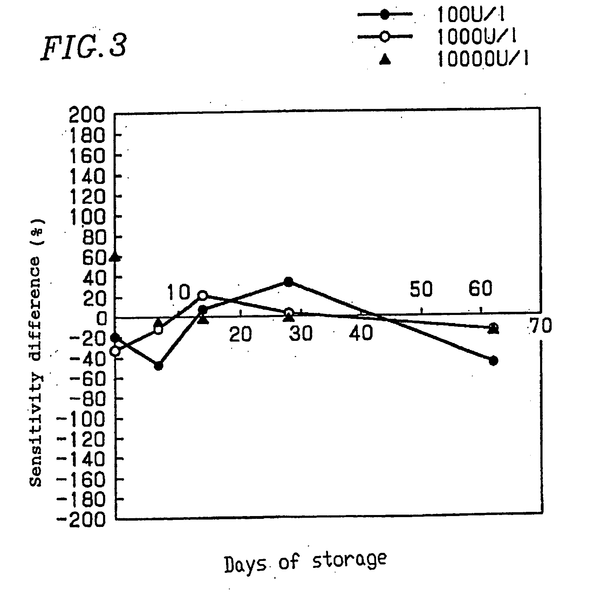

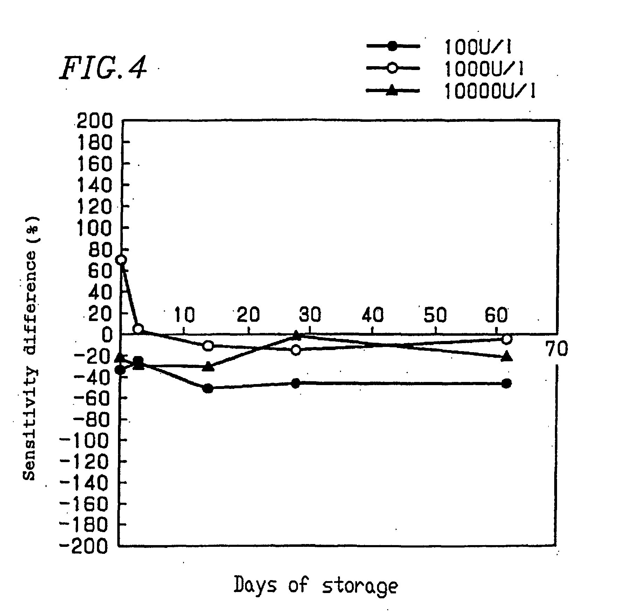

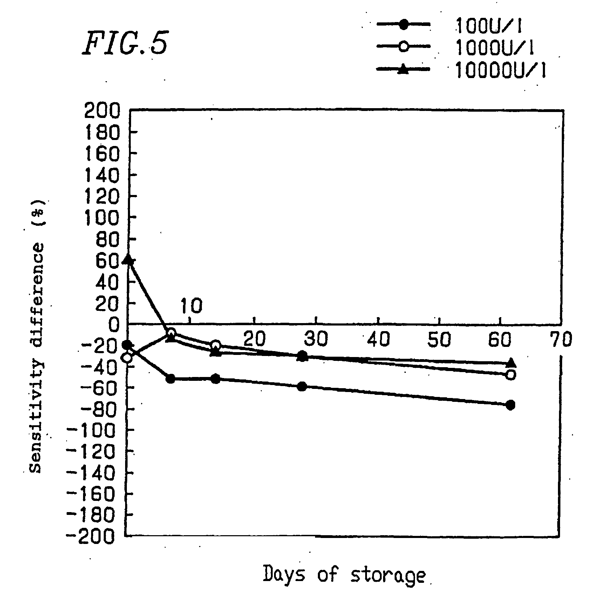

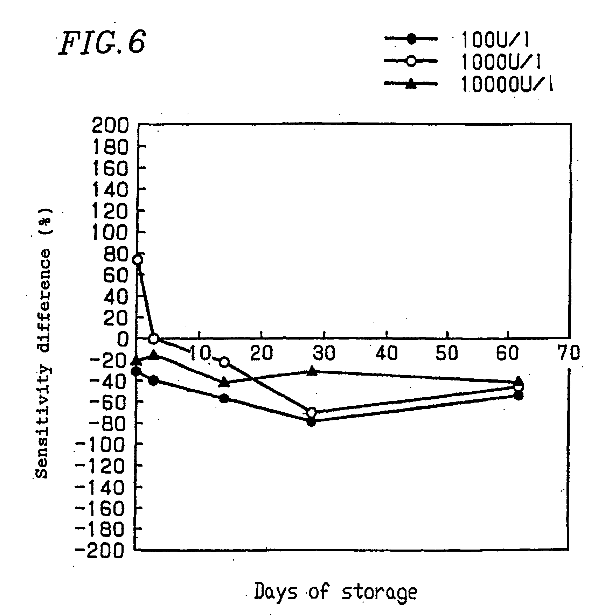

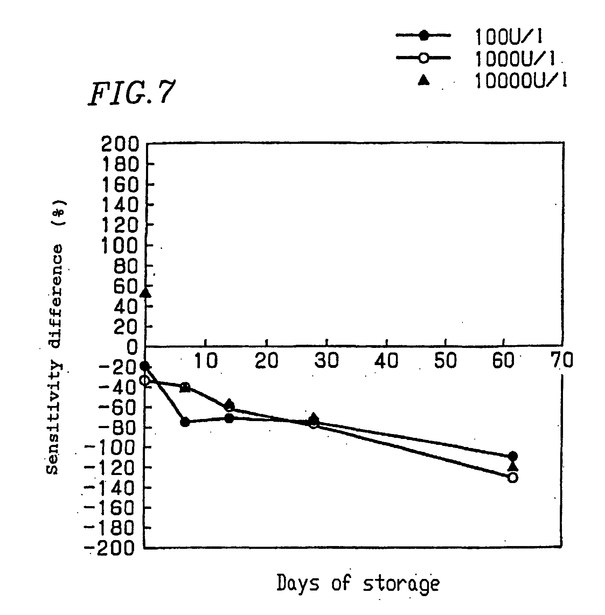

Figure **2** shows the storage stability at 4°C of the immunochromatographic device of Example 1. Figure **3** shows the storage stability at 4°C of the immunochromatographic device of Comparative Example 1. Figure **4** shows the storage stability at 25°C of the immunochromatographic device of Example 1. Figure 5 shows the storage stability at 25°C of the immunochromatographic device of Comparative Example 1. Figure **6** shows the storage stability at 40°C of the immunochromatographic device of Example 1. Figure 7 shows the storage stability at 40°C of the immunochromatographic device of Comparative Example 1. In Figures **2** to **7**, the horizontal axis represents the number of days of storage after production of an immunochromatographic device until performance of measurement, while the vertical axis represents the sensitivity difference between hCG concentration obtained from reflectance absorbance at each measurement and the actual hCG concentration of the sample solution based on a hCG concentration-reflectance absorbance calibration curve prepared immediately after the production of the device. Filled circles indicate data for the sample solution having a concentration of 100 U/l, open circles indicate data for the sample solution having a concentration of 1000 U/l, and triangles indicate data for the sample solution having a concentration of 10000 U/l.

As can be seen from the figures, in the immunochromatographic device of Comparative Example 1, the greater the number of days of storage, the lower the sensitivity. Such tendency is more significant as the storage temperature is increased. In contrast to this, the immunochromatographic device of Example 1 showed changes in its sensitivity at any of the storage temperatures from the day of the production until day 14, and substantially stable sensitivity after day 14 until day 62. In the immunochromatographic device of Example 1, the storage stability at 40°C was maintained for one month from day 14 in a storage stability acceleration test shown at 40°C in Figure **6**. Therefore, about 1 to 1.5-year storage stability can be guaranteed where the above-described period is converted to storage stability at room temperature. Moreover, precision can be guaranteed in terms of a variation in CV value.

### (Example 2) Optimization of sucrose concentration

An immunochromatographic device was produced in the same manner as that of Example 1, except that in the step of preparing a gold colloid sensitized anti-hCG-α antibody solution, 0, 1, 3, 5, 7 or 10% sucrose was added to the gold colloid sensitized anti-hCG-α antibody solution under respective conditions.

### (Preparation of sample solution)

hCG solution having a known concentration was added to serum to adjust the concentration thereof to 0.01, 0.1, and 1 mg/dl.

### (Quantitative determination of hCG concentration)

The immunochromatographic devices carrying respective sucrose concentrations in Example 2 were stored at 40°C for 30 days. The measurement results of the immunochromatographic devices were assessed immediately after the production of the devices and 30 days after production so as to determine an optimum sucrose concentration. According to the results of Example 1 (Figures 2 to 7), if a degradation in sensitivity after storage at 40°C for one month can be suppressed within around -20%, 1.5-year storage stability at 4°C and 25°C can be guaranteed.

In measurement, 40 µl of 0.01, 0.1 or 1 mg/dl hCG solution was added to the sample introduction region **10** of the prepared immunochromatographic device and was allowed to develop on the membrane. Quantification of the hCG concentration of a sample solution was performed after 5 minutes by measuring a coloration in the determination region **12** by reflectance absorbance at 520 nm using a reflection absorption spectrophotometer (manufactured by Shimadzu: CS9300).

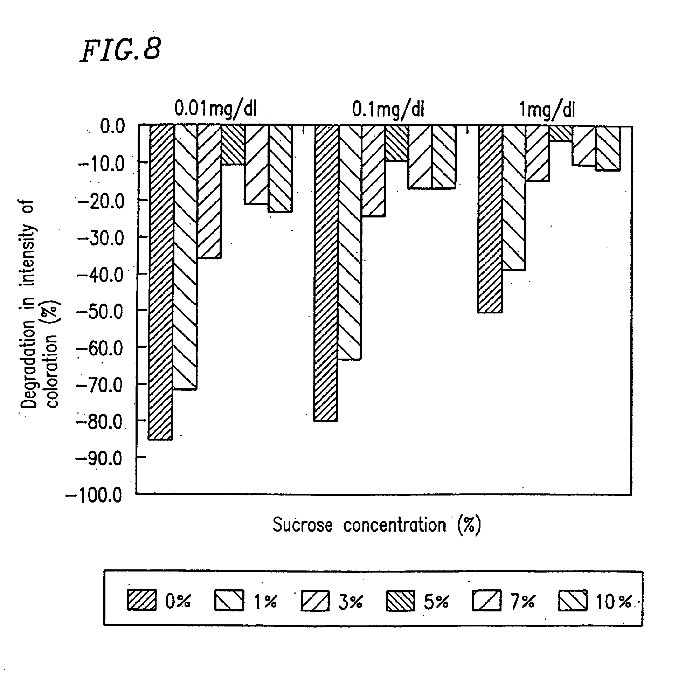

Figure **8** shows the degradation rate of the immunochromatographic device of Example 2 after storage at 40°C for one month where the intensity of a coloration immediately after the production of the device is used as a reference. According to the above-described results, the device having a sucrose concentration of no less than 3% could achieve a coloration whose intensity could be suppressed by only around -20% despite harsh conditions, i.e., storage at 40°C.

In Example 2, since the specimen is serum, it has a certain level of viscosity which is expected to have an adverse influence on the precision of quantitative determination. Therefore, it is preferable that sugar concentration is as low as possible within a range in which the degradation of the intensity of a coloration can be suppressed.

### (Example 3) Performance of storage stability test

Next, immunochromatographic devices for measuring hCG, some having a sugar concentration of 5% and the others containing no sugar, were prepared and subjected to a real-time storage stability test. The test is shown in Example 3.

The immunochromatographic devices of Example 3 were produced in the same manner as that of Example 2, except that in the step of adjusting the gold colloid sensitized anti-hCG-α antibody solution, sucrose was added to the solution to 5% or no sucrose was added. One of the thus-produced devices which did not contain sucrose in its labeling region was used as a Comparative Example, while another which contained 5% w/v sucrose was used as an Example. These devices will be described in detail below.

### (Quantitative determination of hCG concentration)

Quantitative determination of hCG concentration in a sample was performed immediately after production of the immunochromatographic device of Example 3 and after 3 days, 7 days, 14 days, 25 days, 52 days, and 80 days of storage after the production. As for the immunochromatographic device of the Comparative Example, quantitative determination was performed immediately after the production of the device and after 3 days, 7 days, 14 days, 26 days, and 54 days of storage after the production. The storage temperature was 25°C.

In measurement, 40 µl of 0.01, 0.1 or 1 mg/dl hCG solution was added to the sample introduction region **10** of the prepared immunochromatographic device and was allowed to develop on the membrane. Quantification of the hCG concentration of a sample solution was performed after 5 minutes by measuring a coloration in the determination region **12** by reflectance absorbance at 520 nm using a reflection absorption spectrophotometer (manufactured by Shimadzu: CS9300).

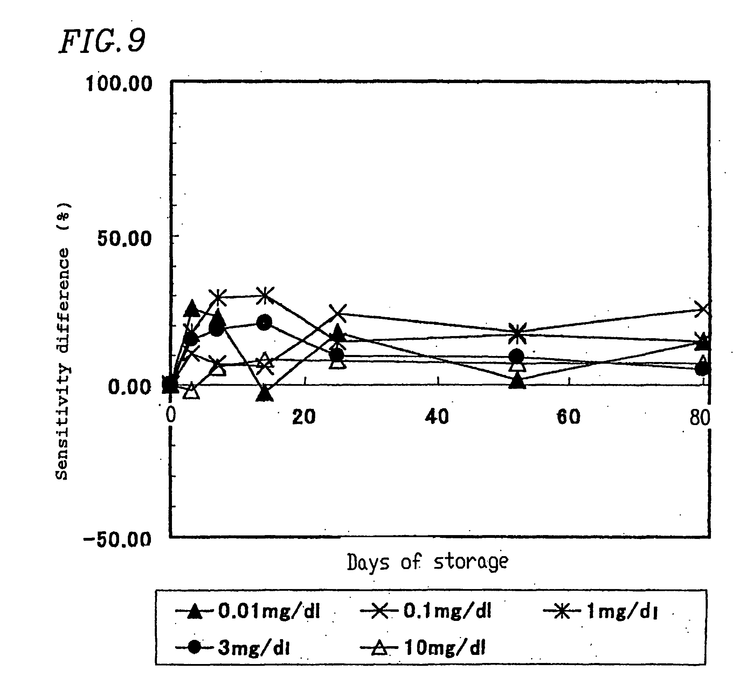

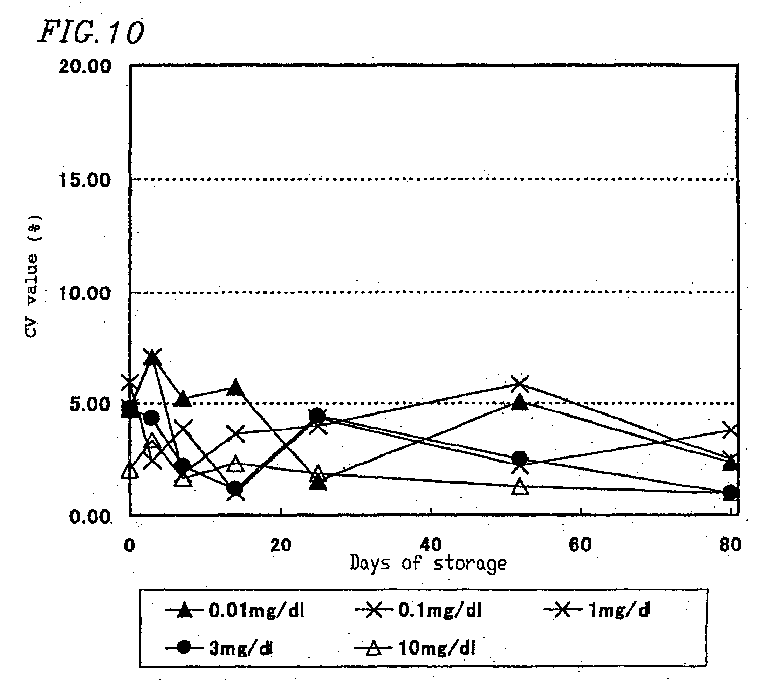

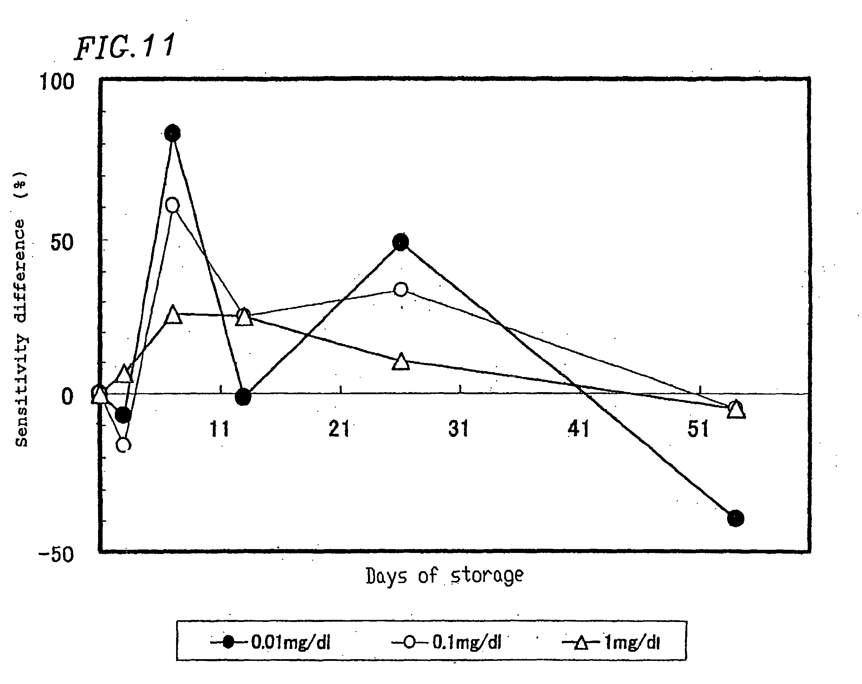

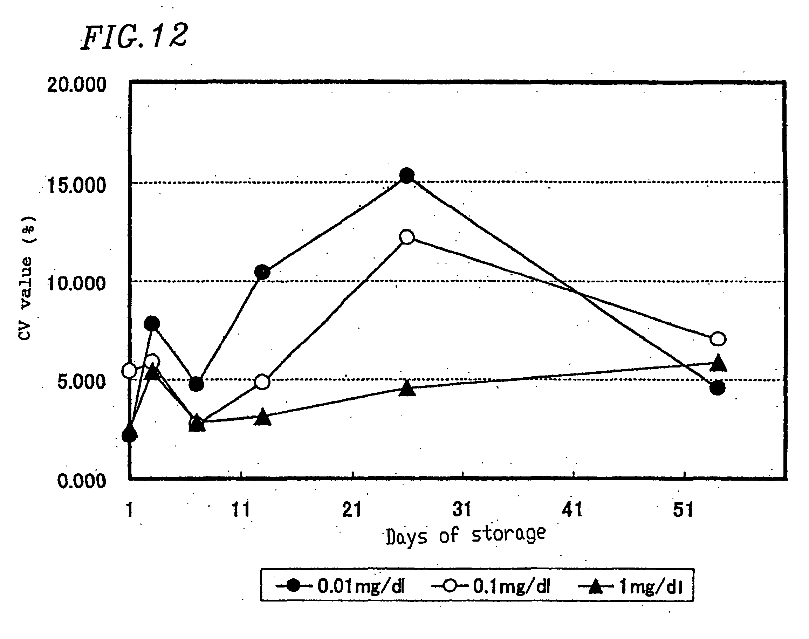

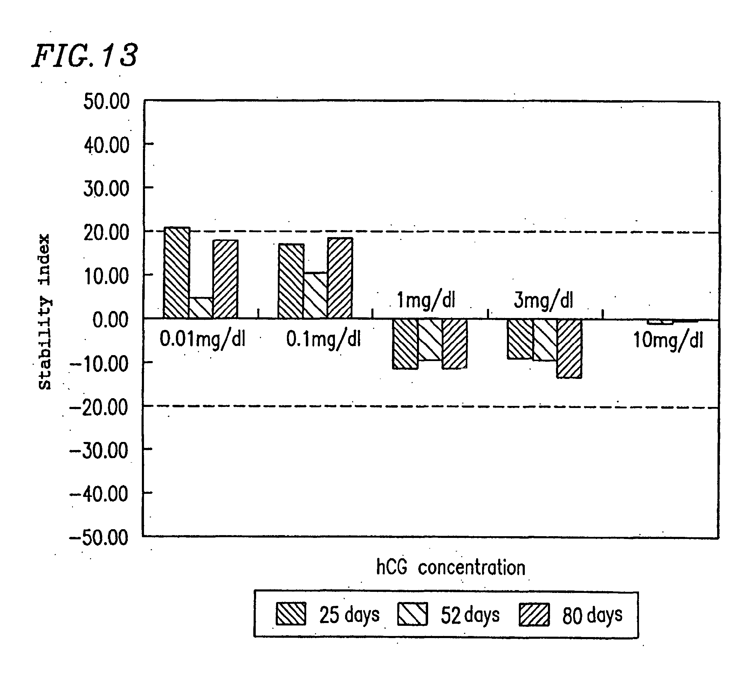

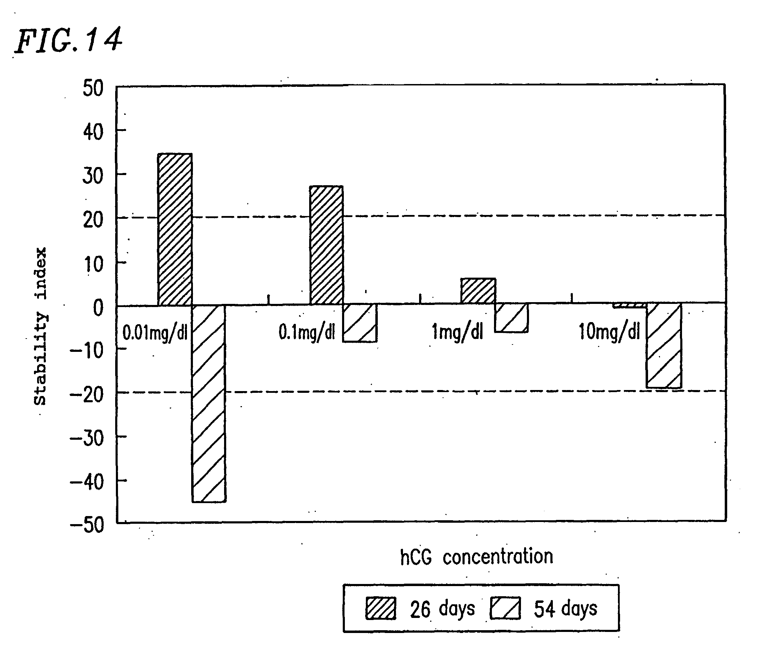

Figure **9** shows the storage stability at 25°C of the immunochromatographic device of Example 3. Figure **10** shows the precision (CV value) of the storage stability at 25°C of the immunochromatographic device of Example 3. Figure **11** shows the storage stability at 25°C of the immunochromatographic device of the Comparative Example. Figure **12** shows the precision (CV value) of the storage stability at 25°C of the immunochromatographic device of the Comparative Example. In Figures **9** and **10**, the horizontal axis represents the number of days of storage after production of an immunochromatographic device until performance of measurement, while the vertical axis represents a degradation rate with respect to the intensity of a coloration immediately after the production of the device. In Figures **11** and **12**, the horizontal axis represents the number of days of storage after production of an immunochromatographic device until performance of measurement, while the vertical axis represents CV value which is an index of the precision of quantification. Further, Figures **13** and **14** show stability indexes representing the degradation rates of Example 3 and the Comparative Example, respectively, where measured values at day 14 were used as a reference.

As can be seen from the figures, in the immunochromatographic device of the Comparative Example, the greater the number of days of storage, the lower the sensitivity. In contrast to this, the immunochromatographic device of Example 3 showed a degradation in its sensitivity from the day of the production until day 14, and substantially stable sensitivity after day 14. Concerning the CV value, whereas the Comparative Example showed a degradation in performance, the immunochromatographic device of Example 3 had a degradation rate within around 5% and the quantification precision was guaranteed.

Particularly, the immunochromatographic device will be discussed regarding the stability index. In Example 3 (Figure **13**), the stability index was maintained within ±20, and there was little variation in sensitivity between measurement results at three time points (days 25, 52 and 80). The possibility that degradation will not occur can be said to be high for subsequent measurements. On the other hand, in the Comparative Example (Figure **14**), although the stability index was maintained within ±20 at the most concentrations, a variation (reduction) of up to 70% at the maximum (0.01 mg/dl) was observed between day 26 and day 54. A similar variation (reduction) tendency is seen for the other concentrations. Therefore, it is predicted that stability index will reach an undesirable range in subsequent measurements. As described above, it was demonstrated that the extracorporeal diagnostic of the present invention provides precise results for a considerably long term.

### INDUSTRIAL APPLICABILITY

According to the present invention, the storage stability of an extracorporeal diagnostic can be improved by adding a sugar or a sugar derivative, even when quantitative determination is performed. The extracorporeal diagnostic of the present invention provides high reliability and ease of handling to the user, and can be utilized not only in clinical tests but also in POC.

## Claims

1. An extracorporeal diagnostic for measuring a substance to be tested in a specimen, comprising:
1) a reagent which specifically binds to the substance to be tested; and
2) a compound comprising at least one hydroxyl group and at least one aldehyde group or ketone group.

2. An extracorporeal diagnostic according to claim 1, wherein the reagent is an antibody or an antigen, or a derivative thereof.

3. An extracorporeal diagnostic according to claim 1, wherein the compound is a sugar or a sugar derivative.

4. An extracorporeal diagnostic according to claim 1; further comprising bovine serum albumin, casein, surfactant, or skim milk.

5. An extracorporeal diagnostic according to claim 1, wherein the compound comprises sugar alcohol or a derivative thereof.

6. An extracorporeal diagnostic according to claim 1, wherein the compound comprises sucrose or sorbitol.

7. An extracorporeal diagnostic according to claim 1, wherein the compound comprises sucrose and sorbitol.

8. An extracorporeal diagnostic according to claim 1, wherein the compound is present at a concentration of no less than about 3 w/v%.

9. An extracorporeal diagnostic according to claim 1, wherein the compound is present at a concentration of about 3 w/v% to about 10 w/v%.

10. An extracorporeal diagnostic according to claim 1, wherein the extracorporeal diagnostic comprises a plurality of reagents which specifically bind to the protein, the plurality of reagents comprise a first antibody or antigen, or a derivative thereof, and a second antibody or antigen, or a derivative thereof.

11. An extracorporeal diagnostic according to claim 1, wherein the reagent is labeled with a colloidal particle, a latex particle, a pigment, a micelle, an enzyme, a fluorescent material, or a phosphorescent material.

12. An extracorporeal diagnostic according to claim 1, wherein the reagent and the compound are retained on a support.

13. An extracorporeal diagnostic according to claim 12, wherein the reagent and the compound are retained on separate regions of the support.

14. An extracorporeal diagnostic according to claim 12, wherein the support is a solid-phase matrix.

15. An extracorporeal diagnostic according to claim 12, wherein the support comprises a porous material.

16. An extracorporeal diagnostic according to claim 12, wherein the support comprises a labeling region, a determination region, and a sample introduction region,
1) the labeling region comprises a first antibody or antigen which binds to the substance to be tested,
2) the determination region comprises a second antibody or antigen which binds to the substance to be tested, and is disposed in fluid communication with the labeling region,
3) the sample introduction region is disposed in fluid communication with the labeling region.

17. An extracorporeal diagnostic according to claim 16, wherein the compound is contained in at least one region selected from the group consisting of the labeling region and the determination region.

18. An extracorporeal diagnostic according to claim 16, wherein the compound is contained in the labeling region.

19. A method for producing an extracorporeal diagnostic for measuring a substance to be tested in a specimen, the method comprising the steps of:
1) providing a reagent which specifically binds to the substance to be tested; and
2) providing a compound comprising at least one hydroxyl group and at least one aldehyde group or ketone group.

20. A method for detecting a substance to be tested in a specimen, the method comprising the steps of:
A) providing an extracorporeal diagnostic, the extracorporeal diagnostic comprising:
1) a reagent which specifically reacts with the substance to be tested; and
2) a compound comprising at least one hydroxyl group and at least one aldehyde group or ketone group;
B) providing the specimen to the extracorporeal diagnostic;
C) disposing the extracorporeal diagnostic under a condition that the reagent specifically reacts with the specimen; and
D) detecting a signal caused by a specific reaction of the reagent with the specimen.

21. Use of an extracorporeal diagnostic for measuring a substance to be tested in a specimen, the extracorporeal diagnostic comprising:
1) a reagent which specifically binds to the substance to be tested; and
2) a compound comprising at least one hydroxyl group and at least one aldehyde.group or ketone group.

## Amended claims

### Amended claims under Art. 19.1 PCT

**1.** (Amended) An extracorporeal diagnostic for measuring a protein in a specimen, comprising:
1) a reagent which specifically binds to the protein; and
2) a compound comprising at least one hydroxyl group and at least one aldehyde group or ketone group, the compound having a concentration of about 3 w/v% to about 10 w/v%,
wherein the reagent and the compound are retained on a support,
the support comprises a labeling region, a determination region, and a sample introduction region,
A) the labeling region comprises a first antibody or antigen which binds to the protein,
B) the determination region comprises a second antibody or antigen which binds to the protein, and is disposed in fluid communication with the labeling region.
C) the sample introduction region is disposed in fluid communication with the labeling region.

**2.** An extracorporeal diagnostic according to claim 1, wherein the reagent is an antibody or an antigen, or a derivative thereof.

**3.** An extracorporeal diagnostic according to claim 1, wherein the compound is a sugar or a sugar derivative.

**4.** An extracorporeal diagnostic according to claim 1, further comprising bovine serum albumin, casein, surfactant, or skim milk.

**5.** An extracorporeal diagnostic according to claim 1,
wherein the compound comprises sugar alcohol or a derivative thereof.

**6.** An extracorporeal diagnostic according to claim 1, wherein the compound comprises sucrose or sorbitol.

**7.** An extracorporeal diagnostic according to claim 1, wherein the compound comprises sucrose and sorbitol.

**8.** (Deleted)

**9.** (Deleted)

**10.** An extracorporeal diagnostic according to claim 1, wherein the extracorporeal diagnostic comprises a plurality of reagents which specifically bind to the protein, the plurality of reagents comprise a first antibody or antigen, or a derivative thereof, and a second antibody or antigen, or a derivative thereof.

**11.** An extracorporeal diagnostic according to claim 1, wherein the reagent is labeled with a colloidal particle, a latex particle, a pigment, a micelle, an enzyme, a fluorescent material, or a phosphorescent material.

**12.** (Deleted)

**13.** (Amended) An extracorporeal diagnostic according to claim 1, wherein the reagent and the compound are retained on separate regions of the support.

**14.** (Amended) An extracorporeal diagnostic according to claim 1, wherein the support is a solid-phase matrix.

**15.** (Amended) An extracorporeal diagnostic according to claim 1, wherein the support comprises a porous material.

**16.** (Deleted)

**17.** (Deleted)

**18.** (Deleted)

**19.** (Amended) A method for producing an extracorporeal diagnostic for measuring a protein in a specimen, the method comprising the steps of:
1) providing a reagent which specifically binds to the protein; and
2) providing a compound comprising at least one hydroxyl group and at least one aldehyde group or ketone group, the compound having a concentration of about 3 w/v% to about 10 w/v%; and
3) causing the reagent and the compound to be retained on a support, wherein the support comprises alabeling region, a determination region, and a sample introduction region,
A) the labeling region comprises a first antibody or antigen which binds to the protein,
B) the determination region comprises a second antibody or antigen which binds to the protein, and is disposed in fluid communication with the labeling region,
C) the sample introduction region is disposed in fluid communication with the labeling region.

**20.** (Amended) A method for detecting a protein in a specimen, the method comprising the steps of:
A) providing an extracorporeal diagnostic, the extracorporeal diagnostic comprising:
1) a reagent which specifically reacts with the protein; and
2) a compound comprising at least one hydroxyl group and at least one aldehyde group or ketone group, the compound having a concentration of about 3 w/v% to about 10 w/v%,
wherein the reagent and the compound are retained on a support,
the support comprises a labeling region, a determination region, and a sample introduction region,
A) the labeling region comprises a first antibody or antigen which binds to the protein,
B) the determination region comprises a second antibody or antigen which binds to the protein, and is disposed in fluid communication with the labeling region,
C) the sample introduction region is disposed in fluid communication with the labeling region;
B) providing the specimen to the extracorporeal diagnostic;
C) disposing the extracorporeal diagnostic under a condition that the reagent specifically reacts with the specimen; and
D) detecting a signal caused by a specific reaction of the reagent with the specimen.

**21.** (Amended) Use of an extracorporeal diagnostic for measuring a protein in a specimen, the extracorporeal diagnostic comprising:
1) a reagent which specifically binds to the protein; and
2) a compound comprising at least one hydroxyl group and at least one aldehyde group or ketone group, the compound having a concentration of about 3 w/v% to about 10 w/v%,
wherein the reagent and the compound are retained on a support,
the support comprises a labeling region, a determination region, and a sample introduction region,
A) the labeling region comprises a first antibody or antigen which binds to the protein,
B) the determination region comprises a second antibody or antigen which binds to the protein, and is disposed in fluid communication with the labeling region,
C) the sample introduction region is disposed in fluid communication with the labeling region.
